# EUROPEAN PATENT APPLICATION

(11) **EP 4 706 735 A1**
(43) Date of publication of application: **11.03.2026**
(21) Application number: 25201177.0
(22) Date of filing: 09.09.2025
(51) Int. Cl.: A61M 25/06, A61M 60/865, A61M 60/13, A61M 60/174, A61M 60/216, A61M 60/414

(54) **SPLIT SLEEVE FOR USE WITH AN INTRAVASCULAR PUMP**

(30) Priority: 10.09.2024 US 202463692844 P
(71) Applicant: Abiomed, Inc., Danvers, MA 01923 (US)
(72) Inventor: MELTON, James, Danvers, MA 01923 (US); MOUX, Jairo Leandro, Danvers, MA 01923 (US); PERKINS, Charles, Danvers, MA 01923 (US); LOUGHLIN, Cam, Danvers, MA 01923 (US)
(74) Representative: Wittmer, Maximilian

(57) **Abstract**

The disclosed technology includes a flexible sleeve having a split that can be removably disposed over an inlet of an intravascular pump while the pump is inserted into a cardiovascular system. The split sleeve can be configured to prevent blood from flowing out the inlet, thereby reducing or preventing blood loss. As the intravascular pump is inserted into an introducer sheath and into a patient, the physician can apply pressure to the split sleeve and the inlet of the intravascular pump, thereby creating a seal and preventing blood loss.

## Description

### CROSS REFERENCE TO RELATED APPLICATIONS

This application claims the benefit of priority under 35 U.S.C. § 119 to prior filed U.S. Provisional Patent Application No. 63/692,844, filed September 10, 2024 (Attorney Docket No.: 266489.000007 (D0273.US01/ABD0273USPSP1)), the entire contents of which is hereby incorporated by reference as if set forth in full herein.

### FIELD

The present invention relates generally to medical devices, and in particular to sleeves used with intravascular pumps to prevent fluid loss.

### BACKGROUND

This invention relates to devices and methods used with a blood pump, in particular an intravascular blood pump for percutaneous insertion into a patient's blood vessel, to support a blood flow in a patient's blood vessel. This invention particularly relates to a right ventricular blood pump to support a blood flow from the vena cava through the right ventricle into the pulmonary artery.

Intravascular blood pumps are inserted into a patient's vessel such as the aorta or vena cava and through a cardiac valve by means of a catheter and can also be referred to as catheter pumps. A blood pump typically comprises a pump section with a blood flow inlet and a blood flow outlet. In order to cause a blood flow from the blood flow inlet to the blood flow outlet, typically an impeller or rotor is rotatably supported within the pump casing about an axis of rotation for conveying blood. The blood pump may be driven by a motor included in the blood pump adjacent to the pump section or may alternatively be driven by a motor outside the patient's body, in which case the motor is connected to the impeller by a flexible drive shaft extending through the catheter.

A right ventricular blood pump is inserted through the inferior or superior vena cava through the right ventricle of a patient's heart into the pulmonary artery by means of a catheter. Typically, the blood flow inlet of the blood pump is placed inside the right atrium, vena cava or right ventricle, while the pump section extends through the tricuspid valve, the right ventricle and the pulmonary valve into the pulmonary artery.

### SUMMARY

The disclosed technology includes a flexible sleeve having a split that can be removably disposed over an inlet of an intravascular pump while the pump is inserted into a cardiovascular system. The split sleeve can be configured to prevent blood from flowing out the inlet, thereby reducing or preventing blood loss. As the intravascular pump is inserted into an introducer sheath and into a patient, the physician can apply pressure to the split sleeve and the inlet of the intravascular pump, thereby creating a seal and preventing blood loss. These and other advantages of the disclosed technology are described further herein.

There is provided, in accordance with the disclosed technology, a sleeve for a catheter. The sleeve comprises a body extending along a longitudinal axis from a proximal end to a distal end. The body of the sleeve includes a distal portion having a first inner diameter and an intermediate portion having a second inner diameter. The second inner diameter can be less than the first inner diameter. The body of the sleeve further includes a slit extending through the body from the proximal end to the distal end.

The disclosed technology includes a medical system comprising an intravascular pump configured for insertion into a heart of a patient and a sleeve. The intravascular pump comprises an inlet and an outlet. The sleeve is configured to be selectively placed about the inlet of the intravascular pump and comprises a body extending along a longitudinal axis from a proximal end to a distal end. The body defines a distal portion having a first inner diameter and configured to extend over the inlet to prevent fluid to flow therethrough and an intermediate portion having a second inner diameter. The second inner diameter can be less than the first inner diameter. The body can further define a slit extending through the body from the proximal end to the distal end.

The disclosed technology further includes a method of performing a medical procedure. The method includes navigating a guidewire to a target location in a heart of a patient and inserting a catheter over the guidewire. The catheter includes an intravascular pump. The method further includes placing a sleeve over an inlet of the intravascular pump and applying pressure to the sleeve over the inlet of the intravascular pump while inserting the intravascular pump into an introducer sheath to prevent fluid from passing through the inlet of the intravascular pump.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements. The figures depict one or more implementations of the inventive devices, by way of example only, not by way of limitation.
FIG. 1 is an illustration of an example intravascular pump inserted into a heart according to aspects of the present disclosure.
FIG. 2 is an illustration of an intravascular pump being inserted into an introducer sheath according to aspects of the present disclosure.
FIG. 3A is a side view of a split sleeve for an intravascular pump according to aspects of the present disclosure.
FIG. 3B is a cross-sectional view of the split sleeve taken along line A-A shown in FIG. 3A, according to aspects of the present disclosure.
FIG. 3C is a cross-sectional view of the split sleeve taken along line B-B shown in FIG. 3A, according to aspects of the present disclosure.
FIG. 3D is a cross-sectional view of the split sleeve taken along line C-C shown in FIG. 3A, according to aspects of the present disclosure.
FIG. 4 is a side view of the split sleeve being disposed over an intravascular pump according to aspects of the present disclosure.
FIG. 5 is another view of the split sleeve being disposed over an intravascular pump according to aspects of the present disclosure.
FIG. 6 is a view of the split sleeve disposed over a catheter with the intravascular pump having been inserted into the introducer sheath according to aspects of the present disclosure.
FIG. 7 is a view of the split sleeve being partially removed from the catheter according to aspects of the present disclosure.
FIG. 8 is a flow chart of a method of using a split sleeve with an intravascular pump according to aspects of the present disclosure.
FIG. 9 is a flow chart of another method of using a split sleeve with an intravascular pump according to aspects of the present disclosure.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

In addition, as used herein, the terms "patient," "host," "user," and "subject" refer to any human or animal subject and are not intended to limit the systems or methods to human use, although use of the subject invention in a human patient represents a preferred embodiment. As discussed herein, "physician" can include a doctor, surgeon, technician, scientist, operator, or any other individual or delivery instrumentation associated with delivery of a multi-electrode catheter for the treatment of drug refractory atrial fibrillation to a subject. As well, the term "proximal" indicates a location closer to the operator or physician whereas "distal" indicates a location further away from the operator or physician.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

Alternative apparatus and system features and alternative method steps are presented in example embodiments herein. Each given example embodiment presented herein can be modified to include a feature and/or method step presented with a different example embodiment herein where such feature and/or step is compatible with the given example as understood by a person skilled in the pertinent art as well as where explicitly stated herein. Such modifications and variations are intended to be included within the scope of the claims.

The disclosed technology relates to a split sleeve configured for insertion over an intravascular pump to prevent fluid, such as blood, from exiting through the pump inlet when the intravascular pump is inserted into a patient. To help the reader better understand the disclosed technology, the intravascular pump will be described first and then the split sleeve will be described in relation to the intravascular pump. It will be appreciated, however, that the disclosed technology can be applicable to other intravascular pumps having differing designs from that described herein. Furthermore, the disclosed technology can be applicable to other catheters in which blood and fluid loss through the catheter when inserting the catheter into a patient is common. Accordingly, the disclosed technology should not be limited to the specific intravascular pump shown in the drawings and described herein.

As an intravascular pump is inserted into the cardiovascular system through an introducer sheath, it is possible for blood to flow through the outlet and out the inlet often causing blood loss. To minimize blood loss, existing systems and methods include a longer sheath outside of the body or faster insertion of the intravascular pump. A longer sheath outside the body, however, limits pump flexibility and increases the risk of over advancing an introducer into the body. Furthermore, inserting the intravascular pump quickly into the body can lead to missed details and potential damage to the cardiovascular system. Accordingly, there is a need for a device and method of preventing or minimizing blood loss during insertion of an intravascular pump into the cardiovascular system. These and other problems are addressed by the technology disclosed herein.

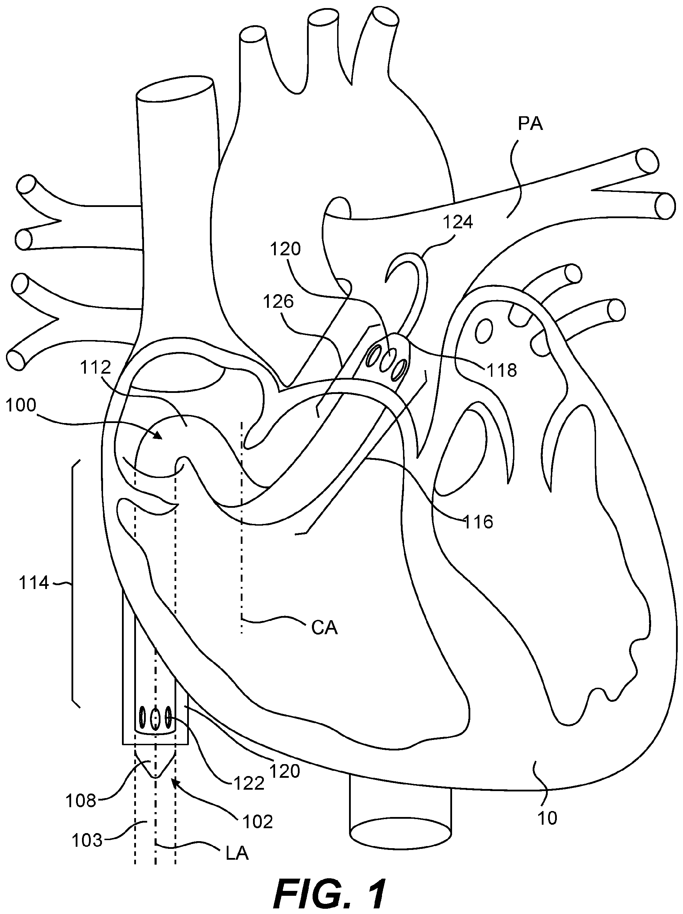

FIG. 1 shows a view of a catheter 100 (sometimes referred to herein as an "intravascular pump" or an "intravascular blood pump") according to certain implementations. The catheter 100 includes a catheter body 103, a pump assembly 108, and a cannula 112. In some embodiments, the cannula 112 may have a pre-formed helical shape. Despite this pre-formed helical shape, the cannula 112 may nevertheless also be flexible, and may thus be capable of straightening (e.g., during insertion over a guidewire), or bending further (e.g., in a patient whose anatomy has tighter dimensions). Further in that regard, cannula 112 may include a shape-memory material configured to allow the cannula 112 to be a different shape (e.g., straight or mostly straight) at room temperatures, and to form the helical shape once the shape- memory material is exposed to the heat of a patient's body. The catheter body 103 extends along a longitudinal axis LA to a distal end 102. The pump assembly 108 is coupled to the distal end 102 of the catheter body 103. The cannula 112 is coupled to a distal end portion 110 of the pump assembly 108. The cannula 112 has a proximal cannula portion 114 and a distal cannula portion 116. The distal cannula portion 116 may have an approximately helical shape. The approximately helical shape has a central axis CA which is substantially parallel to the longitudinal axis 106. The proximal cannula portion 114 may be approximately parallel to the longitudinal axis 106 of the catheter body 103 and may be approximately straight. In some embodiments, the cannula 112 may not have an approximately helical shape and instead may be relatively straight during insertion and capable of bending to accommodate patients' anatomy once the pump is positioned in the patient.

The helically-shaped distal cannula portion 116 includes a partial rotation about the central axis CA. The helical shape of the distal cannula portion 116 may approximate the anatomy of the right heart, allowing the catheter 100 to be inserted into the right heart of a patient with a screw-like motion. A distal tip 126 of the distal cannula portion 116 may deviate from the helical shape in order to further approximate the right heart anatomy. For example, the distal tip 126 may be angled toward the central axis CA i.e., having a greater curvature relative to the remainder of the distal cannula portion 116. In certain implementations, the distal tip 126 may have less of a curvature relative to the remainder of the distal cannula portion 116. In certain implementations, the distal tip 126 of the cannula 112 may narrow toward the distal tip 126 of the distal cannula portion 116, which can further facilitate passage through the heart valves. In certain implementations, a flexible extension 124 can be connected to the distal tip 126 to prevent traumatic contact of the distal tip 126 with interior walls of the heart following insertion. In some embodiments, the catheter 100 may not include a flexible extension 124.

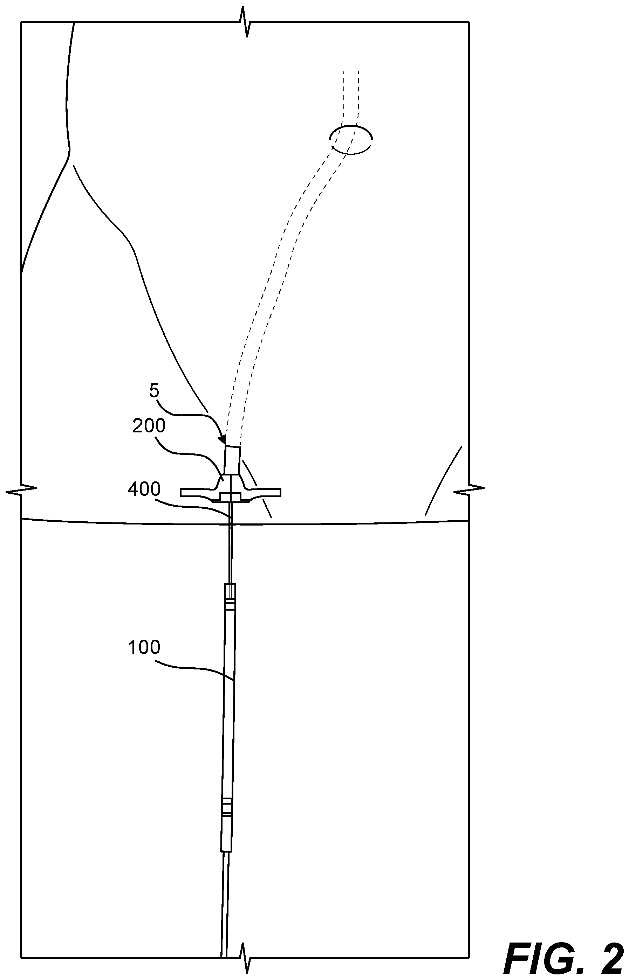

With reference to FIG. 1 and FIG. 2, the catheter 100 can be advanced into the right heart in the following manner. First, an insertion tube 200 is inserted into a femoral vein of a patient at a puncture site 5. The distal tip 126 of the cannula 112 is then inserted through an introducer sheath 200 of the insertion tube and into a femoral vein. In some examples, a guide wire 400 can be inserted into the introducer sheath 200 and navigated to a predetermined location within the patient's heart 10. A catheter 100 can then be inserted over the guide wire 400 and into the introducer sheath 200. The cannula 112 can be advanced through the introducer sheath 200 and into the inferior vena cava into the pulmonary artery. Next, the catheter 100 may be rotated (e.g., clockwise) and advanced in a screw motion through the tricuspid valve toward the pulmonary artery. Then, the screw motion may be repeated to advance the distal tip 126 through the pulmonary valve into the pulmonary artery. When the distal tip 126 of the cannula 112 is positioned within the pulmonary artery, the pump assembly 108 may remain in the inferior vena cava. The pump assembly 108 may be activated once this position is achieved to pump blood from the inferior vena cava into the pulmonary artery. Thus, by enabling the advancing of the cannula 112 by a screw motion, the shape of the distal cannula portion 116 facilitates correct positioning of the catheter 100 in the right side of the heart.

In some examples, the catheter 100 may be inserted via the internal jugular (IJ) vein. For example, in such embodiments, the catheter 100 may be inserted via a catheterization procedure through the IJ vein, into the superior vena cava, through the right atrium, across the pulmonary valve, and into the pulmonary artery. Once positioned in this way, the catheter 100 may deliver blood from inlet ports 122, which sits inside the superior vena cava, through cannula 112, to the outlet ports 120, which sits inside the pulmonary artery.

The cannula 112 is sized to be positioned such that the cannula 112 traverses the inferior vena cava, right atrium, tricuspid valve, right ventricle and pulmonary valve of the heart. This allows inlet ports 122 to be positioned in the inferior vena cava when outlet ports 120 are positioned in the pulmonary artery. In some implementations, the length of the cannula 112 is 17 cm. In certain implementations, the length of the cannula is 10 cm, 12, cm, 15 cm, 17 cm, 18 cm, 20 cm or any other suitable length. The cannula 112 is constructed to allow fluid to flow into the inlet ports 122, through the cannula 112, and out the outlet ports 120. The fluid may be propelled through the cannula 112 by a rotor located in the pump assembly 108. The cannula 112 may be configured to be relatively stiff in order to increase the stability of the cannula 112 once in place in the right heart. The curvature of the cannula 112 may reduce the need for flexibility of the cannula 112 during insertion.

The cannula 112 is also sized for passage through a femoral vein and other vasculature of a patient, such as the internal jugular vein as mentioned herein. In some implementations, the cannula 112 has a cannula diameter of about 22 Fr. In certain implementations, the cannula 112 has a cannula diameter of 7 Fr, 8 Fr, 10 Fr, 11 Fr, 12 Fr, 18 Fr, 20 Fr, 24 Fr, or any other suitable diameter. The cannula diameter may be approximately constant along the length of the cannula 112.

The distal cannula portion 116 of the cannula 112 helps the cannula 112 to be positioned within the right heart of a patient. The helical shape of the distal cannula portion 116 allows the cannula 112 to be dynamically positioned in the right heart using a screw-like rotation motion that follows the anatomy of the right heart. The distal cannula portion 116 may have a curvature that mimics the anatomical pathway of the cannula 112 during positioning in the right heart. In some implementations, the distal cannula portion 116 has a length of about two-thirds of the total length of the cannula 112.

The distal tip 126 of the cannula 112 further facilitates insertion of the cannula 112. The distal tip 126 may deviate from the helix approximating the shape of the remainder of the distal cannula portion 116. In particular, the distal tip 126 of the cannula 112 may have either a greater or lesser curvature than the remainder of the distal cannula portion 116. The curvature of the distal tip 126 causes the distal tip 126 to be oriented toward the tricuspid valve during initial insertion of the cannula 112 into the right atrium. This facilitates insertion because, once in the right atrium, the cannula 112 must make a relatively tight turn toward the tricuspid valve to enter the right ventricle. The shape of the distal tip 126 also causes the distal tip 126 to be oriented toward the pulmonary valve upon entry into the right ventricle. This facilitates insertion because, once in the right ventricle, the cannula 112 must be oriented toward the pulmonary valve to enter the pulmonary artery. In some implementations, the distal tip 126 of the cannula 112 narrows toward the distal end 118 of the distal cannula portion 116, which can further facilitate passage through the heart valves.

The pump assembly 108 may contain a rotor (not shown) which may be driven by an implantable or external drive unit. The pump assembly 108 comprises a housing which may be comprised of a different material than the cannula 112 or catheter body 103. The rotor of the pump assembly 108 may be located in the housing and attached to a drive shaft. Although an embodiment with an implantable motor is shown in FIG. 1, in some implementations the unit driving the drive shaft is located external to the patient's body and the drive shaft extends through the catheter body 103. In some implementations, a motor driving the drive shaft is enclosed within the pump assembly 108. As will be appreciated, any suitable pump and/or drive known may be used.

The pump assembly 108 is configured to provide a fluid flow into the cannula 112 at the inlet ports 122, through the cannula 112, and out the outlet ports 120. The pump assembly 108 may be configured to provide a flow rate of 4 liters per minute (lpm) or more within the right heart of a patient. In some implementations, the pump assembly 108 provides a flow rate of 3 lpm, 3.5 Ipm, 4 Ipm, 4.5 Ipm 5 lpm, 6 Ipm or any other suitable flow rate. In some implementations, the flow rate is chosen based on the needs of the patient.

In some instances, when the catheter 100 is inserted into the introducer sheath 200 and into the femoral vein, blood in the patient can flow into the outlet ports 120, back through the catheter 100 through the cannula 112, and out the inlet ports 122. As will be appreciated, if blood is permitted to flow back through the inlet ports 122 before the catheter 100 has been inserted to an extent that the inlet ports 122 have been inserted through the introducer sheath 200, blood may escape from the patient through the inlet ports 122. To help prevent the loss of blood and other fluid through the inlet ports 122 when the catheter 100 is inserted into the patient, the disclosed technology includes a split sleeve 500 that can be inserted over the catheter 100, and more specifically over the inlet ports 122 to form a seal thereby preventing blood and other fluid from escaping through the inlet ports 122.

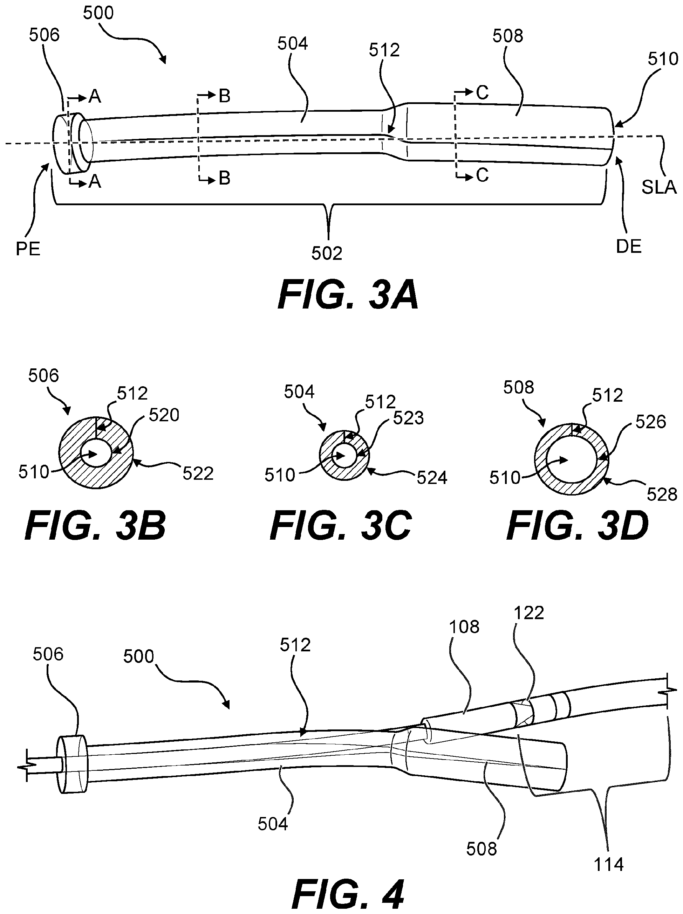

Turning now to FIGs. 3A-7, the split sleeve 500 will be described in greater detail. As shown, the split sleeve 500 includes a body 502 extending along a sleeve longitudinal axis SLA from a proximal end (PE) to a distal end (DE). The body 502 is flexible and configured to conform to the catheter 100 when placed at least partially over the catheter 100. The body 502 can, for example, be formed from a continuous flexible biocompatible material such as silicone or a polyurethane such that a physician can place the split sleeve 500 over, and remove the split sleeve 500 from, the catheter 100. However, as will be appreciated, any suitable material may be used to form the body.

The split sleeve 500 includes an intermediate portion 504 disposed between a proximal portion 506 and a distal portion 508. The split sleeve 500 can include a lumen 510 and a slit 512 which both extend longitudinally through the body 502 along the entire length of the split sleeve 500 from the proximal end to the distal end. In other words, the lumen 510 and the slit 512 can extend through the proximal portion 506, the intermediate portion 504, and the distal portion 508. In this way, the split sleeve 500 can be configured such that a physician can flex open the slit 512 and place the split sleeve 500 over, or remove from, the catheter 100, as shown in FIGs. 4-7.

FIG. 3B is a cross-sectional view of the split sleeve 500 taken along line A-A shown in FIG. 3A, FIG. 3C is a cross-sectional view of the split sleeve 500 taken along line B-B shown in FIG. 3A, and FIG. 3D is a cross-sectional view of the split sleeve 500 taken along line C-C shown in FIG. 3A. As shown in FIG. 3B, the proximal portion 506 includes a proximal portion inner diameter 520 and a proximal portion outer diameter 522. The proximal portion inner diameter 520 can be sized to receive the catheter body 103 and form an interference fit with the catheter 100. For example, the proximal portion inner diameter 520 can be approximately 5 Fr, 6, Fr, 7 Fr, 8 Fr, 9 Fr, 10 Fr, 11 Fr, 12 Fr, 13 Fr, 14 Fr, or 15 Fr depending on the particular application. Similarly, as shown in FIG. 3C, the intermediate portion 504 can have an intermediate portion inner diameter 523 and an intermediate portion outer diameter 524. The intermediate portion inner diameter 523 can be approximately equal to the proximal portion inner diameter 520 while the intermediate portion outer diameter 524 can be less than the proximal portion outer diameter 522. In this way, the intermediate portion 504 can be configured to receive the catheter body 103 but the body 502 can also form a portion on which the physician can grip or push against (e.g., the physician can use the outwardly-extending proximal portion 506 to grip and/or slide the split sleeve 500 longitudinally).

Furthermore, as shown in FIG. 3D, the distal portion 508 can have a distal portion inner diameter 526 and a distal portion outer diameter 528. The distal portion inner diameter 526 can be greater than the proximal portion inner diameter 520 and the intermediate portion inner diameter 523 and be sized to receive the pump assembly 108. For example, the distal portion inner diameter 526 can be approximately 22 Fr. In certain implementations, the distal portion inner diameter 526 can be approximately 7 Fr, 8 Fr, 10 Fr, 11 Fr, 12 Fr, 18 Fr, 20 Fr, or 24 Fr depending on the particular application. That is, the split sleeve 500 can be configured to fit over an end of the pump assembly 108 and extend along a proximal end of the pump assembly 108 along the catheter body 103, thereby forming a seal over the proximal end of the pump assembly 108. In particular, the split sleeve 500 can be configured to extend over the inlet ports 122 of the pump assembly 108 to form a seal over the inlet ports 122. In this way, the split sleeve 500 can be configured to prevent blood or other fluid from flowing back through the inlet ports 122 and escaping to the environment.

In some examples, the split sleeve 500 can be configured such that distal portion 508 alone forms a sufficient seal over the inlet ports 122. In other examples, a physician can apply pressure on the distal portion 508 over the inlet ports 122, thereby preventing fluid from escaping through the inlet ports 122. One of skill in the art will appreciate that the split sleeve 500 can help prevent patient blood loss and the resultant disruption to the sterile environment necessary in an operating room.

The distal portion outer diameter 528 can be sized to form an interference fit with the introducer sheath 200. In this way, when the physician inserts the catheter 100 into the introducer sheath 200 with the split sleeve 500 placed over the pump assembly 108, the split sleeve 500 can be forced to split away from the catheter 100 and removed from the catheter 100. Thus, as the physician inserts the pump assembly 108 into the introducer sheath 200, the split sleeve 500 is naturally caused to dislodge from the pump assembly 108 and permit the pump assembly 108 to be inserted into the introducer sheath 200. This can help ensure the split sleeve 500 provides the beneficial sealing function it is designed for.

In some examples, the distal portion outer diameter 528 and the proximal portion outer diameter 522 are both greater than the intermediate portion outer diameter 524. The distal portion outer diameter 522 and the proximal portion outer diameter 522 can be equal or the distal portion outer diameter 528 can be less than the proximal portion outer diameter 522. In other examples, the distal portion outer diameter 528 can be greater than the proximal portion outer diameter 522 depending on the particular configuration.

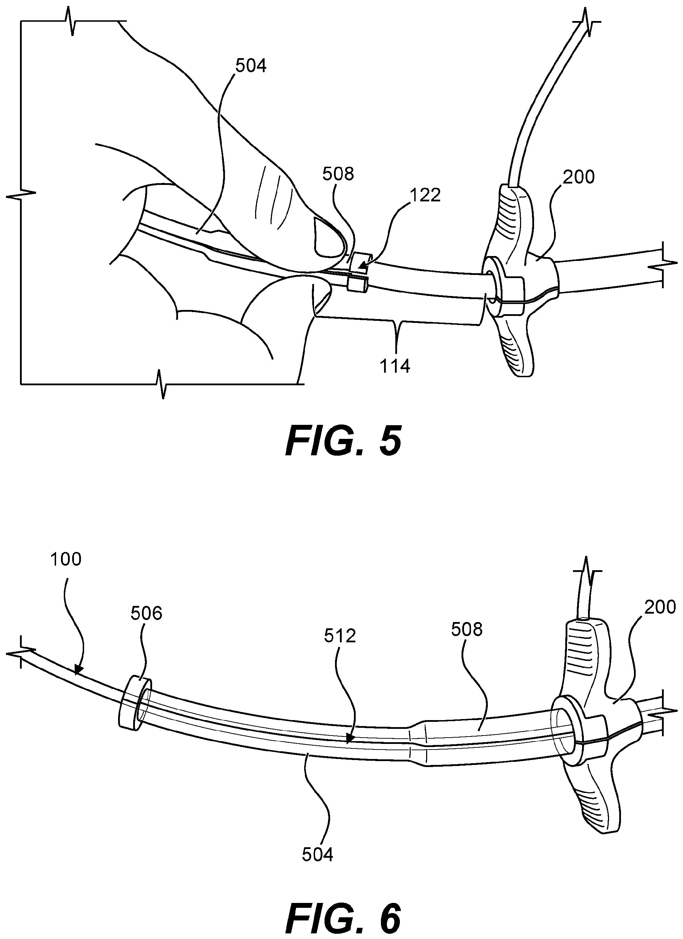

Turning now to FIGs. 4-7, the process of installing, using, and removing the split sleeve 500 will now be described. As shown in FIG. 4, the split sleeve 500 can be aligned with the pump assembly 108 and the catheter body 103, flexed open at the slit 512, and placed over the catheter body 103 and the pump assembly 108. The split sleeve 500 can then be gripped by a physician at least on the distal portion 508 (which is aligned over the inlet ports 122) and used to push the catheter 100 into the introducer sheath 200 for insertion into a patient (as shown in FIG. 5). That is, the split sleeve 500 can be used as both a means to prevent blood loss while also functioning as a convenient tool to grip the catheter 100 when inserting the catheter 100. In some examples, the outer surface of the split sleeve 500 can be textured to help make it easier for a physician to grip the split sleeve 500. Furthermore, in some examples. The split sleeve 500 can be transparent or translucent to enable a physician to see the pump assembly 108, the inlet ports 122, the catheter body 103, and/or blood or other fluid within the split sleeve 500.

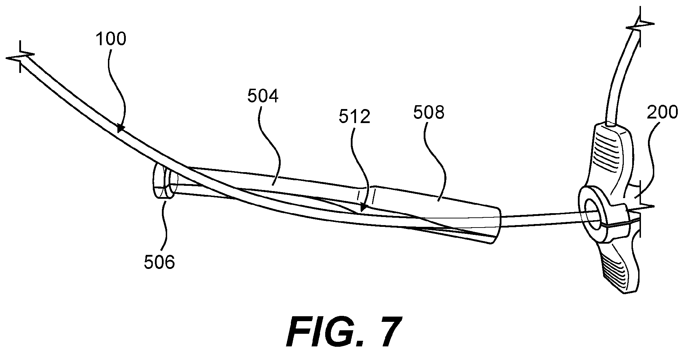

As the catheter 100 is inserted through the introducer sheath 200, the split sleeve 500 can eventually contact the introducer sheath 200. The split sleeve 500 is configured such that the split sleeve 500 is prevented from passing through the introducer sheath 200 and, therefore, becomes dislodged from the pump assembly 108 as the pump assembly 108 is inserted through the introducer sheath 200. With the pump assembly 108 properly inserted into the introducer sheath 200, the split sleeve 500 can be safely removed from the catheter 100. As shown in FIG. 7, the split sleeve 500 can be flexed open at the slit 512 and removed from the catheter 100. In some examples, it may be desirable to leave the split sleeve 500 at least partially attached over the catheter body 103 so that the split sleeve 500 is readily available.

As will be appreciated, the split sleeve 500 can also be used for removing the catheter 100 from the patient through the introducer sheath 200. For example, as the pump assembly 108 is pulled out of the patient through the introducer sheath 200, the split sleeve 500 can be placed over the pump assembly 108 and the catheter body 103 to prevent blood or other fluid from escaping. The split sleeve 500 can be flexed open, placed over the catheter body 103, and aligned in a position to receive the pump assembly 108 and aligned over the inlet ports 122 to prevent blood or other fluid from escaping.

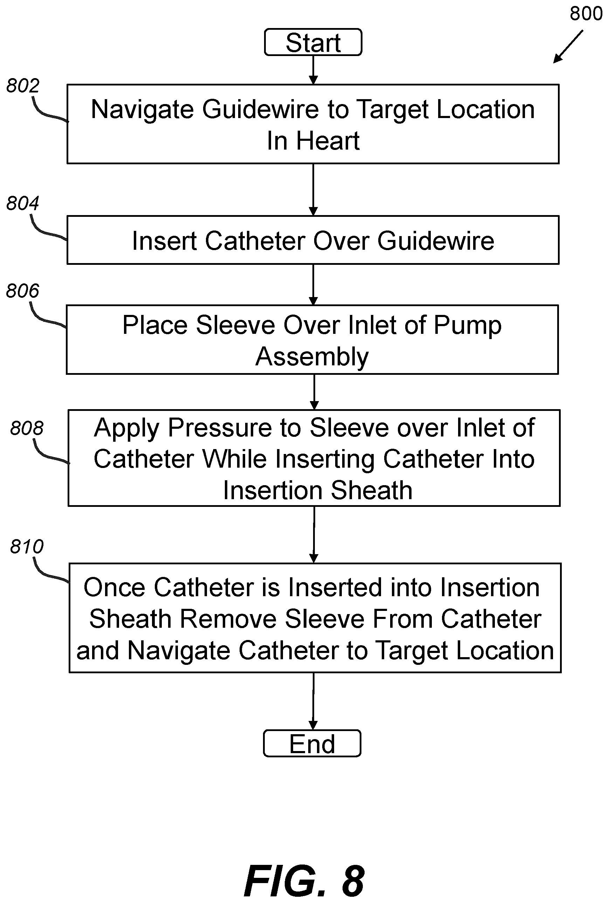

FIG. 8 illustrates an example method 800 of using the split sleeve 500 described herein for insertion of a catheter 100 into a patient. The method 800 can include navigating 802 a guide wire to a target location within a patient (e.g., a target location within a heart of a patient), inserting 804 a catheter (e.g., catheter 100 described herein) over the guidewire, and placing 806 a sleeve (e.g., split sleeve 500 described herein) over the inlet of the catheter (e.g., over inlet ports 122). The method 800 can further include applying 808 pressure to the sleeve over the inlet of the catheter while inserting the catheter into an introducer sheath. For example, as described above, the method 800 can include the physician pinching or otherwise applying pressure to the distal portion 508 over the inlet ports 122 to create a seal and thereby prevent blood or other fluid from escaping. Once the catheter is inserted into the introducer sheath (e.g., once the pump assembly 108 has been inserted into the introducer sheath 200), the method 800 can further include removing 810 the sleeve from the catheter and navigating the catheter to a target location.

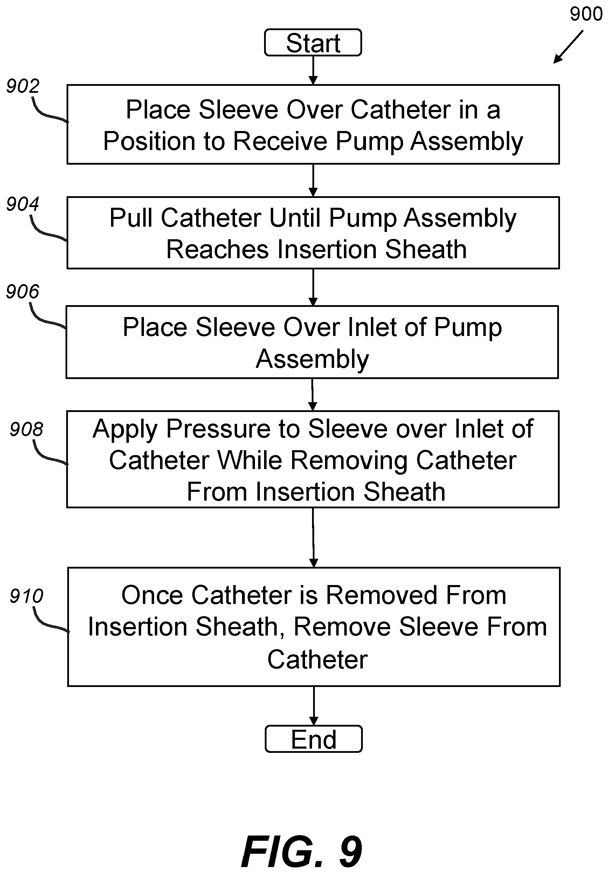

As shown in FIG. 9, the disclosed technology can further include a method 900 of using a split sleeve 500 during removal of a catheter 100. The method 900 can include placing 902 a split sleeve over a catheter in a position to receive a pump assembly (e.g., placing the split sleeve 500 over a catheter body 103 in a position to receive the pump assembly 108), pulling 904 the catheter until the pump assembly reaches the introducer sheath, and placing 906 the sleeve over the inlet (e.g., inlet ports 122) of the pump assembly. The method 900 can further include applying 908 pressure on the sleeve over the inlet of the catheter while removing the catheter from the introducer sheath. Once the catheter is removed from the introducer sheath, the method 900 includes removing 910 the sleeve from the catheter.

The disclosed technology can be further understood according to the following clauses:
- Clause 1:: A sleeve (500) for a catheter (100), the sleeve comprising: a body (502) extending along a longitudinal axis (LA) from a proximal end (PE) to a distal end (DE), the body defining: a distal portion (508) having a first inner diameter; an intermediate portion (504) having a second inner diameter, the second inner diameter being less than the first inner diameter; and a slit (512) extending through the body from the proximal end to the distal end.
- Clause 2:: The sleeve of clause 1, wherein the body comprises a flexible biocompatible material.
- Clause 3:: The sleeve of clause 1 or clause 2, wherein the body is configured to flex open at the slit for the sleeve to receive the catheter or to be removed from the catheter.
- Clause 4:: Clause 4: The sleeve of any one of the preceding clauses, wherein the catheter comprises an intravascular blood pump (100).
- Clause 5:: The sleeve of clause 4, wherein the sleeve is configured to extend over an inlet (122) of the intravascular pump to prevent fluid from passing through the inlet.
- Clause 6:: The sleeve of any one of the preceding clauses, wherein the body comprises a contiguous polymer material.
- Clause 7:: The sleeve of clause 4, wherein the body comprises at least one of a silicone or a polyurethane.
- Clause 8:: The sleeve of any one of the preceding clauses, wherein the distal portion comprises a first outer diameter and the intermediate portion comprises a second outer diameter, the second outer diameter being less than the first outer diameter.
- Clause 9:: The sleeve of clause 8, wherein the body further defines a proximal portion (506) disposed proximate the intermediate portion and having an inner diameter approximately equal to the second inner diameter.
- Clause 10:: The sleeve of clause 9, wherein the proximal portion comprises a third outer diameter, the third outer diameter being greater than the second outer diameter.
- Clause 11:: The sleeve of clause 10, wherein the third outer diameter is less than the first outer diameter.
- Clause 12:: The sleeve of any one of the preceding clauses, wherein the body comprises a textured outer surface.
- Clause 13:: A medical system comprising: an intravascular pump (100) configured for insertion into a heart (10) of a patient, the intravascular pump comprising an inlet (122) and an outlet port (120); and a sleeve (500) configured to be selectively placed about the inlet of the intravascular pump, the sleeve comprising a body (502) extending along a longitudinal axis (LA) from a proximal end (PE) to a distal end (DE), the body defining: a distal portion (508) having a first inner diameter and configured to extend over the inlet to prevent fluid to flow therethrough; an intermediate portion (504) having a second inner diameter, the second inner diameter being less than the first inner diameter; and a slit (512) extending through the body from the proximal end to the distal end.
- Clause 14:: The medical system of clause 13, wherein the body comprises a flexible biocompatible material.
- Clause 15:: The medical system of clause 13 or clause 14, wherein the body is configured to flex open at the slit for the sleeve to receive at least a portion of the intravascular pump or to be removed from the intravascular pump.
- Clause 16:: The medical system of any one of clauses 13-15, wherein the body comprises a contiguous polymer material.
- Clause 17:: The medical system of clause 16, wherein the body comprises at least one of a silicone or a polyurethane.
- Clause 18:: The medical system of any one of clauses 13-17, wherein the distal portion comprises a first outer diameter and the intermediate portion comprises a second outer diameter, the second outer diameter being less than the first outer diameter.
- Clause 19:: The medical system of clause 18, wherein the body further defines a proximal portion (506) disposed proximate the intermediate portion and having an inner diameter approximately equal to the second inner diameter.
- Clause 20:: The medical system of clause 19, wherein the proximal portion comprises a third outer diameter, the third outer diameter being greater than the second outer diameter.
- Clause 21:: The medical system of clause 20, wherein the third outer diameter is less than the first outer diameter.
- Clause 22:: The medical system of any one of clauses 13-21, wherein the body comprises a textured outer surface.
- Clause 23:: A method (800) of performing a medical procedure comprising: navigating a guidewire to a target location in a heart of a patient; inserting a catheter over the guidewire, the catheter comprising an intravascular pump; placing a sleeve over an inlet of the intravascular pump; and applying pressure to the sleeve over the inlet of the intravascular pump while inserting the intravascular pump into an introducer sheath to prevent fluid from passing through the inlet of the intravascular pump.
- Clause 24:: The method of clause 23 further comprising navigating the intravascular pump to the target location.
- Clause 25:: The method of clause 23 or clause 24, wherein placing a sleeve over the inlet of the intravascular pump comprises flexing the sleeve open at a slit of the sleeve and extending the sleeve over at least a portion of the intravascular pump.
- Clause 26:: The method of any of clauses 23-25 further comprising removing the sleeve from the intravascular pump.
- Clause 27:: The method of clause 26, wherein removing the sleeve from the intravascular pump comprises flexing the sleeve open at a slit of the sleeve and removing the sleeve from the intravascular pump.
- Clause 28:: The method of clause 26, wherein removing the sleeve from the intravascular pump comprises advancing the catheter along with the sleeve until the sleeve contacts the introducer sheath, the introducer sheath causing the sleeve to split as the catheter is advanced into the introducer sheath.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the spirit of the disclosure or equivalent to the inventions found in the claims, it is the intent that this disclosure will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A sleeve for a catheter, the sleeve comprising:
a body extending along a longitudinal axis from a proximal end to a distal end, the body defining:
a distal portion having a first inner diameter;
an intermediate portion having a second inner diameter, the second inner diameter being less than the first inner diameter; and
a slit extending through the body from the proximal end to the distal end.

2. The sleeve of claim 1, wherein the body comprises a flexible biocompatible material.

3. The sleeve of claim 1 or claim 2, wherein the body is configured to flex open at the slit for the sleeve to receive the catheter or to be removed from the catheter.

4. The sleeve of any one of the preceding claims, wherein the catheter comprises an intravascular blood pump.

5. The sleeve of claim 4, wherein the sleeve is configured to extend over an inlet of the intravascular pump to prevent fluid from passing through the inlet.

6. The sleeve of any one of the preceding claims, wherein the body comprises a contiguous polymer material.

7. The sleeve of claim 4, wherein the body comprises at least one of a silicone or a polyurethane.

8. The sleeve of any one of the preceding claims, wherein the distal portion comprises a first outer diameter and the intermediate portion comprises a second outer diameter, the second outer diameter being less than the first outer diameter.

9. The sleeve of claim 8, wherein the body further defines a proximal portion disposed proximate the intermediate portion and having an inner diameter approximately equal to the second inner diameter.

10. The sleeve of claim 9, wherein the proximal portion comprises a third outer diameter, the third outer diameter being greater than the second outer diameter.

11. The sleeve of claim 10, wherein the third outer diameter is less than the first outer diameter.

12. The sleeve of any one of the preceding claims, wherein the body comprises a textured outer surface.

13. A medical system comprising:
an intravascular pump configured for insertion into a heart of a patient, the intravascular pump comprising an inlet and an outlet port; and
a sleeve according to any one of the preceding claims, the sleeve being configured to be selectively placed about the inlet of the intravascular pump.

14. The medical system of claim 13, wherein the body is configured to flex open at the slit for the sleeve to receive at least a portion of the intravascular pump or to be removed from the intravascular pump.
